# EUROPEAN PATENT SPECIFICATION

(11) **EP 3 772 350 B1**
(45) Date of publication and mention of the grant of the patent: **31.08.2022**
(21) Application number: 20189465.6
(22) Date of filing: 04.08.2020
(51) Int. Cl.: A61F 2/46, A61B 5/00, A61B 5/107, A61B 5/11, A61B 5/1495, A61B 17/00, A61B 17/02, A61B 90/00

(54) **SYSTEM TO DETERMINE CERVICAL IMPLANT SIZE**
SYSTEM ZUR BESTIMMUNG DER GRÖSSE EINES HALSIMPLANTATS
SYSTÈME POUR DÉTERMINER LA TAILLE D'UN IMPLANT CERVICAL

(30) Priority: 05.08.2019 US 201962882909 P
(43) Date of publication of application: 10.02.2021
(73) Proprietor: Zimmer Biomet Spine, Inc., Westminster, Colorado 80021 (US)
(72) Inventor: PERMESWARAN, Vijay N., Iowa (US); GANDHI, Anup, Colorado, 80027 (US)
(74) Representative: Taor, Simon Edward William

(56) References cited:
- WO-A1-2010/065545
- US-A1- 2013 079 680
- US-B2- 9 216 098
- US-B2- 9 693 882

## Description

### TECHNICAL FIELD

This document pertains generally, but not by way of limitation, to devices, instruments, and methods used during spinal surgical procedures, such those involving implantation of cervical prosthetic replacement discs. More specifically, but not by way of limitation, the present application relates to instruments for measuring the intervertebral disc space and selecting a properly sized prosthesis for implantation.

### BACKGROUND

Spinal deformity correction often involves implantation of a prosthetic device to replace damaged or missing vertebral disc material. For example, in the cervical spine Zimmer Biomet provides a device marketed under the Mobi-C^{®} name that enables disc replacement while maintaining native range of motion. The Mobi-C^{®} device is designed to replicate the natural mobility provided by a patient's vertebral disc material. One important step in the disc replacement procedure is sizing the intervertebral disc space to select the proper size of prosthetic disc. Prosthetic systems, such as Mobi-C^{®} from Zimmer Biomet, often include a set of trialing instruments that replicate the prosthesis sizes for use in the procedure. Trialing instruments, typically, mimic the overall dimensions of each prosthesis size with an attached handle for insertion and removal. The trialing process often involves multiple additional x-ray exposures for the patient to allow a surgeon to ensure proper vertebral endplate coverage as well as height. Typically, available trialing instruments are tools for use by a surgeon, but still require judgment and experience from the surgeon to obtain a proper fit.

US9216098 B2 discloses a method and apparatus for making a size measurement within an intervertebral space by placing an expandable and contractible device into the intervertebral space, expanding the device, measuring a size characteristic of the space, contracting the device and then removing it. The measurement may be accomplished by an external x-ray or other imaging device imaging the expanded device in situ or by mechanically operated devices. An expansion and contraction mechanism such as fluid containing bladder or mechanically shifted members expands the device which later contracts in a controlled manner to the contracted size. An apparatus and method is provided for the measuring of the intervertebral space at a controlled distraction force. The apparatus includes an expandable device for providing a measurement within the intervertebral space and facilitating the measurement of the angulations of the lordotic curve of the intervertebral space..

WO2010065545 discloses a method and apparatus for making a size measurement within an intervertebral space by placing an expandable and contractible device into the intervertebral space, expanding the device, measuring a size characteristic of the space, contracting the device and then removing it. The measurement may be accomplished by an external x-ray or other imaging device imaging the expanded device in situ or by mechanically operated devices. An apparatus and method is provided for the measuring of the intervertebral space at a controlled distraction force. The apparatus includes an expandable device for providing a measurement within the intervertebral space and facilitating the measurement of the angulations of the lordotic curve of the intervertebral space.

### SUMMARY

According to an aspect, there is provided an implant-trialing instrument as set out in claim 1. Optional features are set out in the dependent claims.

The methods discussed in this specification are of an explanatory nature only. The claimed invention is directed to an implant-trialing instrument.

### OVERVIEW

The present inventors have recognized, among other things, that a problem to be solved can include the prevention of the use of over-sized intervertebral implants. Research conducted by the present inventors indicates that a properly sized Mobi-C^{®} implant maintains range of motion in 1 and 2 level procedures that is nearly identical to native intact spinal levels. However, selecting too tall of an implant by as little as 1mm can produce a 15% drop in range of motion across the cervical spine (C2 to T1) and over a 50% drop in range of motion at the corrected level. The research shows that overstuffing effectively creates a quasi-fusion at the replacement level. Currently available trialing instruments fail to provide any objective measures or output to assist in guiding a surgeon in selecting a properly sized implant (prosthesis). The present inventors have developed a trialing instrument that can provide objective feedback to assist a surgeon in selecting the proper implant size. In an example, the trialing instrument can include an expandable trialing mechanism with a built-in force/pressure sensor, which in combination provide measurement of disc space at known distraction pressures. The following specification further details instruments and methods for minimizing the loss of range of motion through proper implant size selection by providing objective measurements of the disc space.

This overview is intended to provide an overview of subject matter of the present patent application. It is not intended to provide an exclusive or exhaustive explanation of the invention. The detailed description is included to provide further information about the present patent application.

### BRIEF DESCRIPTION OF THE DRAWINGS

FIG. 1A is a block diagram illustrating components of an adjustable trialing instrument, in accordance with at least one example of the disclosure.
FIG. 1B is a perspective view illustrating a prototype adjustable trialing instrument, in accordance with at least one example of the disclosure.
FIG. 1C is a perspective view illustrating an example expandable trialing mechanism, in accordance with at least one example of the disclosure.
FIG. 1D is an end view illustrating an example proximal end of a prototype adjustable trialing instrument, in accordance with at least one example of the disclosure.
FIGS. 2A-2D are illustrations depicting example external user interfaces, in accordance with at least one example of the disclosure.
FIG. 3 is a schematic diagram of a robotic surgical system, in accordance with at least one example of the disclosure.
FIG. 4A is a flow chart illustrating operation of a technique for operating an implant sizing instrument, in accordance with at least one example of the disclosure.
FIG. 4B is a flow chart illustrating operations of a surgical technique for determining a proper implant size using an adjustable trialing instrument, in accordance with at least one example of the disclosure.
FIG. 5 is a block diagram of an example machine upon which any one or more of the techniques discussed herein may perform in accordance with some embodiments.

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

### DETAILED DESCRIPTION

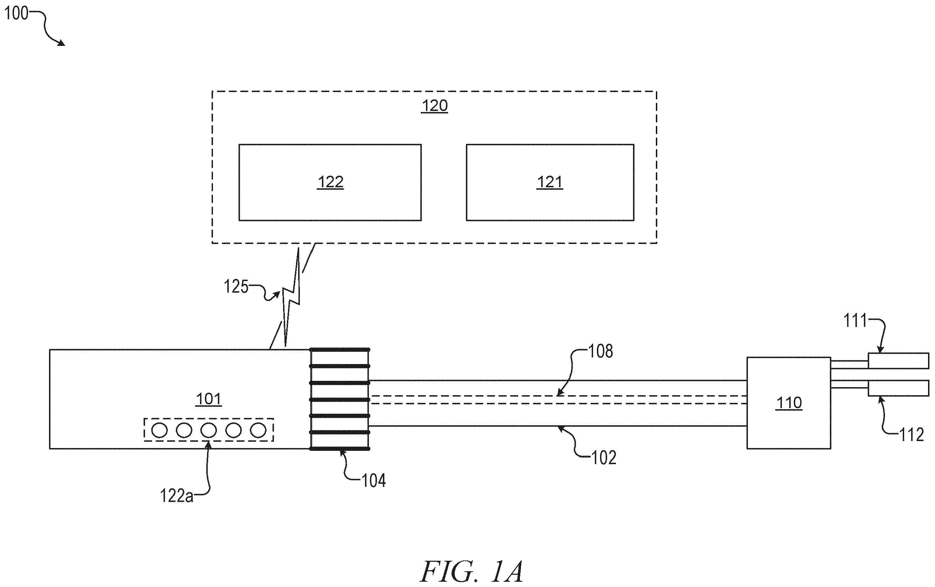

FIG. 1A is a block diagram illustrating components of an adjustable trialing instrument, in accordance with at least one example of the disclosure. In this example, the adjustable trialing instrument 100 can include components such as a handle assembly 101, an elongate extension 102, an adjustable implant trial mechanism 110, and a control module 120. The control module 120 can include a control circuit 121 and a user interface 122 connected to other parts of the adjustable trialing instrument via a communication link 125. In some examples, the control module 120 can be integrated into the handle assembly 101 and/or along the elongate extension 102. The control circuit 121 can be embedded into the handle assembly 101 with the communication link 125 running the length of the elongate extension 102. The user interface 122 can include simple LEDs or a small display. In some examples, the user interface 122 can be separate from the adjustable trial instrument 100, such as on a mobile device (e.g., mobile phone or tablet) with a wireless communication connection to the control module 120.

In certain examples, the user interface 122 can include a series of light emitting diodes (LEDs) integrated into the handle assembly 101. The LEDs can provide indication of when a force or pressure sensor within the adjustable trial mechanism 110 is outputting a measurement within a pre-defined range. For example, the user interface 122 can include three LEDs, one red, one yellow, and one green. The red LED can indicate pressures that are significantly out of tolerance either high or low. The yellow LED can indicate pressure measurements that are marginally out of tolerance either high or low. The green LED can indicate pressure measurements within tolerance. In another example, the user interface 122 can include five (5) LEDs arranged in a sequence of Red, Yellow, Green, Yellow, Red, providing additional information to the user (shown in FIG. 1A as user interface 122a). In this example, the first Red and Yellow LEDs indicate low out of tolerance pressure measurements and the second Yellow and Red LEDs indicate high out of tolerance pressure measurements.

In an example, the handle assembly 101 can include an adjustment mechanism mechanically coupled to the adjustable trial mechanism 110 through the elongate extension 102. The adjustment mechanism can include a dial, wheel or rotatable collar type adjustment mechanism integrated into the handle assembly 101. In an example, a portion of the handle assembly 101 can include a cylindrical adjustment collar, such as adjustment collar 104, coupled via gearing or similar mechanism to a shaft 108 that rotates with the rotation of the adjustment collar 104. In certain examples, the internal gearing can increase the mechanical advantage between the adjustment collar 104 and the shaft 108. Rotation of the adjustment collar 104 can transfer rotation to the shaft 108 that can then rotate gearing within the adjustable trial mechanism 110 to expand or contract the distraction paddles, such as superior paddle 111 and inferior paddle 112, within an intervertebral space. Another example is illustrated in FIGs. IB-1D that includes a dial-type adjustment mechanism (see adjustment dial 105).

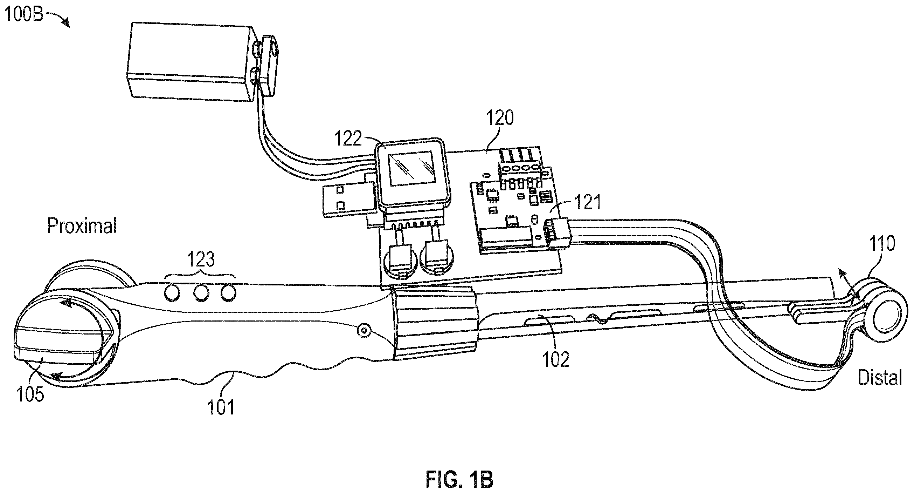

FIG. 1B is a perspective view of a prototype adjustable trialing instrument, in accordance with at least one example of the disclosure. In this example, the implant sizing instrument 100 includes a handle assembly 101, an elongate extension 102, an adjustment dial 105, an adjustable trialing mechanism 110, and a control module 120. The control module 120 includes a control circuit 121 and a user interface display 122. This example also illustrates an alternative user interface including an LED fit indicator 123. The adjustment dial 105 can be rotated to open and close the adjustable trialing mechanism 110. As discussed above, rotational input to the adjustment dial 105 is transferred through the handle assembly 101 and elongate extension 102 to the adjustable trialing mechanism 110. In this example, the user interface 122 is a small external display that can be integrated into the handle assembly 101. The user interface 122 can display a graphical depiction of force/pressure being measured by a sensor integrated into the adjustable trialing mechanism. For prototyping purposes, a FlexiForce sensor and OEM (original equipment manufacturer) development kit was utilized from Tekscan, Inc. (https://www.tekscan.com/products-solutions/electronics/flexiforce-oem-development-kit). The FlexiForce sensor is a resistance-based force sensor designed to detect between 0 and 4.4 Newtons (N) of force. If different force ranges are needed for a particular application, sensors are readily available for 0-111 N and 0-445 N, among other ranges. The OEM development kit includes a control circuit using a signal conditioning circuit as outlined in the documentation included with the kit.

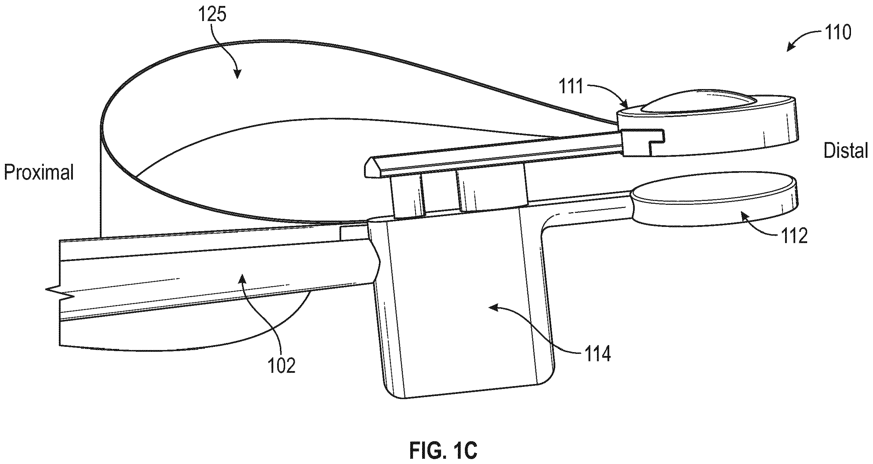

FIG. 1C is a perspective view of the adjustable trialing mechanism 110, in accordance with at least one example of the disclosure. In this example, the adjustable trialing mechanism 110 includes a superior paddle 111, an inferior paddle 112, and an expansion mechanism 114. The expansion mechanism 114 can receive rotational input through the elongated extension 102 to expand or contract the distraction paddles including the superior paddle 111 and inferior paddle 112. Thus, the expansion mechanism 114 can comprise a mechanism for converting rotational movement to linear movement, such a worm gear mechanism and the like. The sensor can be disposed within the superior paddle 111 and is connected to the control module 120 via a wired communication link 125 in this example. In other examples, the sensor can communicate wirelessly over a protocol such as Bluetooth LE (low energy), or similar low energy protocol. Alternatively, in some examples, the sensor can be communicatively coupled to the control module 120 via a wired connection run through the elongate extension 102.

In certain examples, the adjustable trialing mechanism 110 is a disposable device attachable to the elongated extension 102. In this example, the adjustable trialing mechanism 110 is inserted onto the end of the elongate extension 102 prior to use, with coupling interface including gearing that mates with exposed gearing on the elongated extension 102.

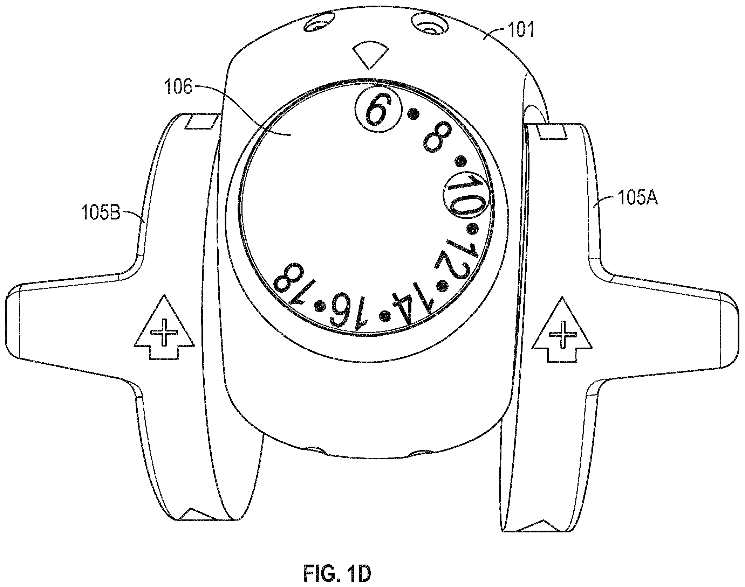

FIG. 1D is a proximal view of an end portion of the handle assembly 101, in accordance with at least one example of the disclosure. In this example, the handle assembly 101 includes two lateral adjustment dials 105A, 105B and an implant size indicator 106. In this example, the implant size indicator 106 is a mechanical rotary indicator that is coupled mechanically to the adjustment dials 105A, 105B and the adjustable trialing mechanism 110. As the adjustment dials 105A, 105B are manipulated, up and down relative to FIG. 1D to adjust pressure/force/distance, the implant size indicator 106 moves, rotationally relative to FIG. 1D, correspondingly to provide indication of the vertical height size indicated by the expansion amount. Accordingly, when the pre-defined pressure measurement is achieved, the surgeon can read the implant size indicator 106 to determine what height implant to utilize in the procedure. In this example, the adjustment dials 105A, 105B are coupled and move when either dial is adjusted.

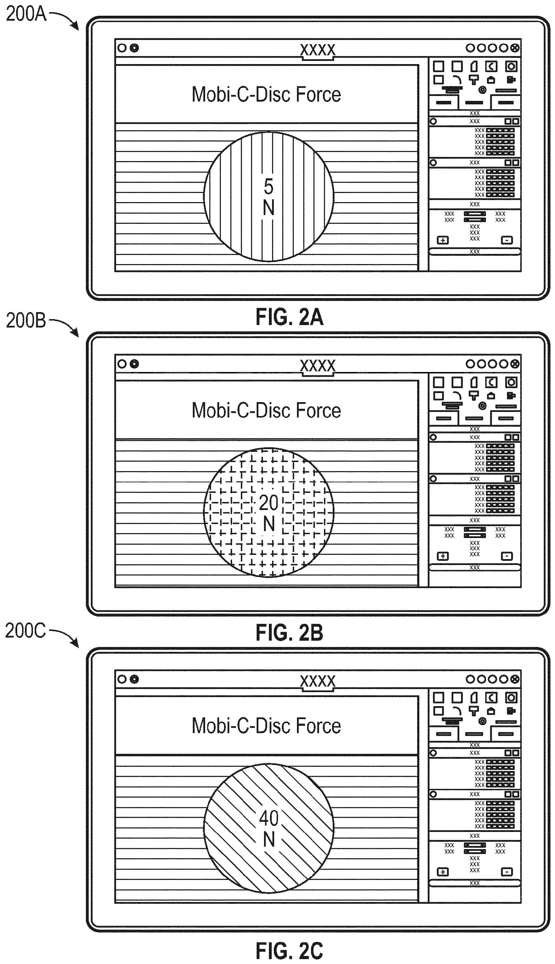

FIGs. 2A-2C are illustrations of an external user interface 200A-200C for the implant sizing instrument 100. In these examples, the user interface 200A-200C is depicted on a display of a computing device wirelessly communicating with the control module 120. The user interfaces 200A-200C are examples of user interface 122 discussed above. The depicted user interface 200A-200C shows a force measurement within a colored circle. The color-coded circle changes color based on how close the measured force is to the target force or pressure within the intervertebral disc space. The target force can be programmed or updated as needed to align with surgeon preferences and evolve as additional data is made available through use of the implant sizing instrument 100, which can include the ability to record quantitative measurements over time. As the implant sizing instrument 100 distracts the intervertebral disc space and measures the distraction force (or pressure) the user interface 200A-200C is constantly updated. In this example, the target force measurement is 40N, which is shown in FIG. 2C inside a green circle providing a quick and easy to read feedback to the surgeon performing the procedure, while FIGs. 2A and 2B show red and yellow, respectively. In this example, the implant sizing instrument 100 can also include an on-instrument user interface, such as user interface 122a discuss above.

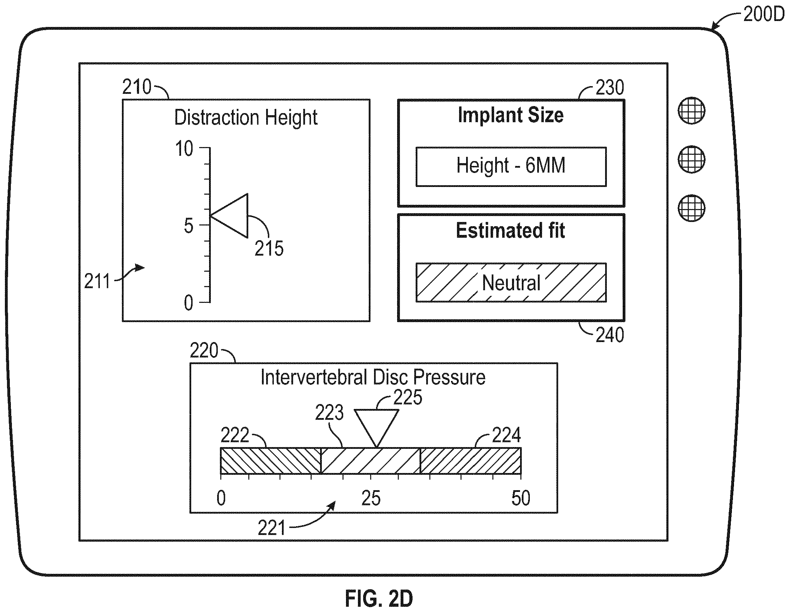

FIG. 2D is a line drawing illustrating a tablet computing device depicting a more detailed user interface 200D, in accordance with at least one example of the disclosure. In this example, the external user interface 200D includes a distraction height component 210, an intervertebral disc pressure component 220, an implant size component 230, and an estimated fit component 240. The distraction height component 210 can include a height scale 211 and a height indicator 215. The distraction height component 210 depicts data representative of the amount of distraction being applied by the implant size instrument 100 via the adjustable trialing mechanism 110. The height indicator 215 moves along the height scale 211 to provide visual feedback on how far the superior paddle 111 is distracted from the inferior paddle 112. The intervertebral disc pressure component 220 depicts real-time information from a force/pressure sensor embedded within one of the distraction paddles (superior paddle 111 or inferior paddle 112). The intervertebral disc pressure component 220 can include user interface elements such as a pressure/force scale 221, a low-pressure range 222, a target pressure range 223, a highpressure range 224, and a pressure indicator 225. As the measured pressure within the distracted intervertebral disc space increases the pressure indicator 225 moves along the pressure scale 221 to visually depict the range of the measurement as well as an actual numerical readout. The external user interface 200D can also include interpreted fields providing an implant size indication and an estimate fit indication. The implant size component 230 includes a field that provides feedback to the surgeon as to the recommended implant height. The recommended implant height is determined based on analysis of the distraction height and intervertebral disc pressure measurements provided by the implant sizing instrument 100. The estimated fit component 240 can provide an additional indication as to how well the recommended implant size is likely to fit within the patient. For example, if the distraction measurement indicates a 6mm implant size, but the intervertebral disc pressure is on the high side of the target range, then the estimate fit component 240 may indicate a tight or neutral-tight fit. In the example depicted in FIG. 2D, the distraction height indicator 215 is just below 6mm and the pressure indicator 225 is in the middle of the target pressure range 223, accordingly the estimated fit component 240 indicates a neutral fit for the selected implant. Again, user interface 200D is an example of the user interface 122 discussed above in reference to FIG. 1.

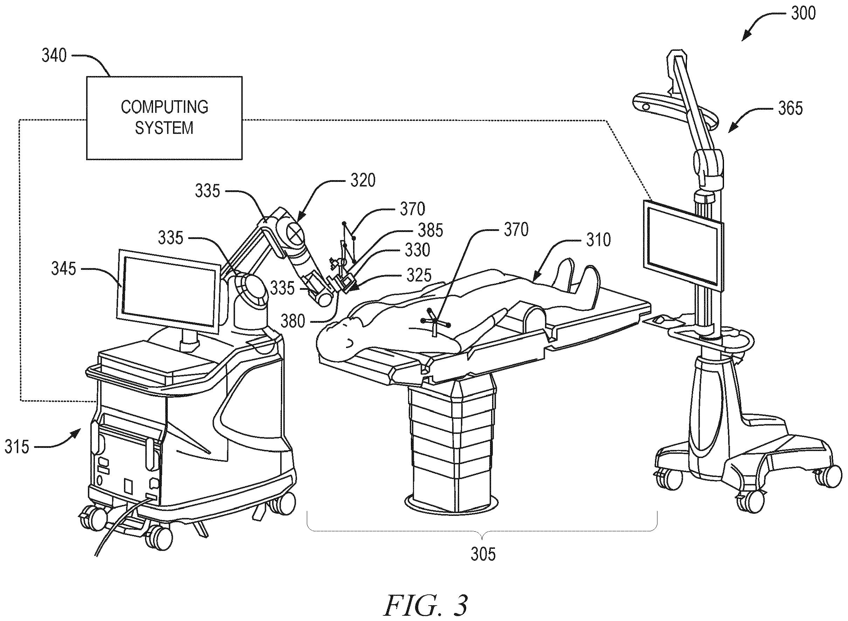

FIG. 3 illustrates surgical system 300 for operation on surgical area 305 of patient 310 in accordance with at least one example of the present disclosure. Surgical area 305 in one example can include a portion of the spine and, in another example, can be an intervertebral disc space in the cervical spine. Surgical area 305 can include any surgical area of patient 310, including but not limited to the shoulder, head, elbow, thumb, spine, and the like, but the discussed examples focus on the cervical spine. Surgical system 300 can also include robotic system 315 with one or more robotic arms, such as robotic arm 320. As illustrated, robotic system 315 can utilize only a single robotic arm. Robotic arm 320 can be a 6 degree-of-freedom (DOF) robot arm, such as the ROSA robot from MedTech, a Zimmer Biomet Holdings, Inc. company. In some examples, robotic arm 320 is cooperatively controlled with surgeon input on the end effector or surgical instrument, such as surgical instrument 325. In other examples, robotic arm 320 can operate autonomously. While not illustrated in FIG. 3, one or more positionable surgical support arms can be incorporated into surgical system 300 to assist in positioning and stabilizing instruments or anatomy during various procedures.

Each robotic arm 320 can rotate axially and radially and can receive a surgical instrument, or end effector, 325 at distal end 330. Surgical instrument 325 can be any surgical instrument adapted for use by the robotic system 315, including, for example, a gripping device such as a pincer grip, a burring device, a reaming device, an impactor device such as a humeral head impactor, a pointer, a probe or the like. In some example, the adjustable trialing instrument 100 can be adapted for use in conjunction with robotic arm 320. Surgical instrument 325 can be positionable by robotic arm 320, which can include multiple robotic joints, such as joints 335, that allow surgical instrument 325 to be positioned at any desired location adjacent or within a given surgical area 305. As discussed below, robotic arm 320 can be used with an adjustable trialing instrument, e.g., adjustable trialing instrument 100 (FIGS. 1A-1D), to measure intervertebral disc space within surgical area 305 to enable computing system 340 to suggest an implant size.

Robotic system 315 can also include computing system 340 that can operate robotic arm 320 and surgical instrument 325. Computing system 340 can include at least memory, a processing unit, and user input devices, as will be described herein. Computing system 340 can also include human interface device 345 for providing images and other objective information obtained by the robotic system 315 for a surgeon to be used during surgery. Computing system 340 is illustrated as a separate standalone system, but in some examples computing system 340 can be integrated into robotic system 315. Human interface device 345 can provide images, including but not limited to three-dimensional images of bones, vertebra, disc spaces, and the like. Human interface device 345 can include associated input mechanisms, such as a touch screen, foot pedals, or other input devices compatible with a surgical environment.

Computing system 340 can receive pre-operative medical images. These images can be received in any manner and the images can include, but are not limited to, computed tomography (CT) scans, magnetic resonance imaging (MRI), two-dimensional x-rays, three-dimensional x-rays, ultrasound, and the like. These images in one example can be sent via a server as files attached to an email. In another example the images can be stored on an external memory device such as a memory stick and coupled to a USB port of the robotic system to be uploaded into the processing unit. In yet other examples, the images can be accessed over a network by computing system 340 from a remote storage device or service.

After receiving one or more images, computing system 340 can generate one or more virtual models related to surgical area 305. Specifically, a virtual model of the anatomy of patient 310 can be created by defining anatomical points within the image(s) and/or by fitting a statistical anatomical model to the image data. The virtual model, along with virtual representations of implants, can be used for calculations related to the desired height, depth, and size of an implant or the like related to be utilized in surgical area 305. In another procedure type, the virtual model can be utilized to determine insertion location, trajectory and depth for inserting an instrument, such as the adjustable trialing instrument 100. The virtual model can also be used to determine bone dimensions, implant dimensions, bone fragment dimensions, bone fragment arrangements, and the like. Any model generated, including three-dimensional models, can be displayed on human interface device 345 for reference during a surgery or used by robotic system 315 to determine motions, actions, and operations of robotic arm 320 or surgical instrument 325. Known techniques for creating virtual bone models can be utilized, such as those discussed in U.S. Patent No. 9,675,461, titled "Deformable articulating templates" or U.S. Patent No. 8,884,618, titled "Method of generating a patient-specific bone shell" both by Mohamed Rashwan Mahfouz, as well as other techniques known in the art.

Computing system 340 can also communicate with tracking system 365 that can be operated by computing system 340 as a stand-alone unit. Surgical system 300 can utilize the Polaris optical tracking system from Northern Digital, Inc. of Waterloo, Ontario, Canada. Tracking system 365 can monitor a plurality of tracking elements, such as tracking elements 370, affixed to objects of interest to track locations of multiple objects within the surgical field. Tracking system 365 functions to create a virtual three-dimensional coordinate system within the surgical field for tracking patient anatomy, surgical instruments, or portions of robotic system 315. Tracking elements 370 can be tracking frames including multiple IR reflective tracking spheres, or similar optically tracked marker devices. In one example, tracking elements 370 can be placed on or adjacent one or more bones of patient 310. In other examples, tracking elements 370 can be placed on robot robotic arm 320, surgical instrument 325, and/or an implant to accurately track positions within a virtual coordinate system associated with surgical system 300. In each instance tracking elements 370 can provide position data, such as patient position, bone position, joint position, robotic arm position, implant position, or the like.

Robotic system 315 can include various additional sensors and guide devices. For example, robotic system 315 can include one or more force sensors, such as force sensor 380. Force sensor 380 can provide additional force data or information to computing system 340 of robotic system 315. Force sensor 380 can be used to monitor impact or implantation forces during certain operations, such as insertion of an implant trial into a vertebral disc space. Monitoring forces can assist in preventing negative outcomes through force fitting components or overstuffing an intervertebral disc space. In other examples, force sensor 380 can provide information on soft-tissue tension in the tissues surrounding a target joint.

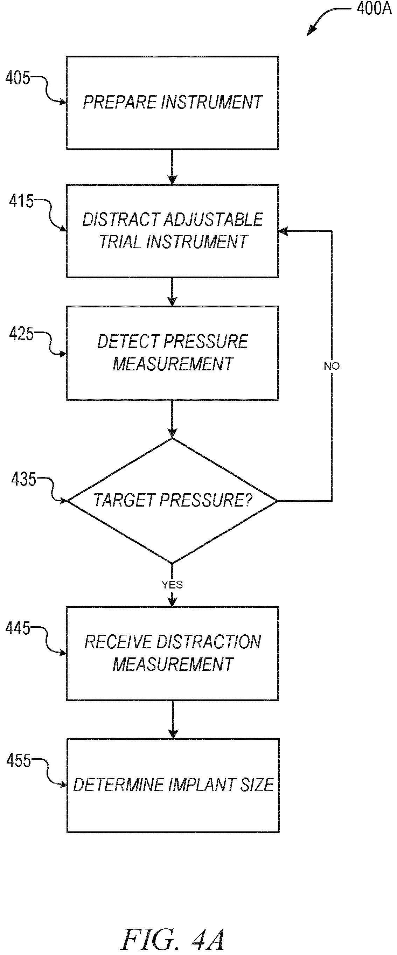

FIG. 4A is a flow chart illustrating operations of a technique for utilizing an implant sizing instrument, in accordance with at least one example of the disclosure. In this example, the technique 400A includes operations such as preparing the instrument at 405, distracting the instrument at 415, detecting pressure measurement at 425, determining whether target pressure has been reached at 435, receiving distraction measurement at 445, and determining implant size at 455. At least portions of the operations in technique 400A can be performed by an external computer system (e.g., tablet or personal computer) or on controller module 120 or surgical system 300.

In an example, the technique 400A can optionally begin at 405 with preparation of the adjustable trialing instrument. Instrument preparation can include operations such a powering up the instrument, resetting or calibrating the pressure/force sensor, connecting the instrument to an external computing device (e.g., establishing a Bluetooth or similar wireless connection), and resetting the adjustable trialing mechanism 110 among other operations. In an example using a disposable adjustable trialing instrument, preparation can include assembly of the adjustable trialing instrument onto an elongate extension. At 415, the technique 400A can continue with a user, such as a surgeon, manipulating an adjustment mechanism on the instrument to distract the distraction paddles. In an example, distracting the distraction paddles allows for measuring a vertical (superior to inferior) distance within an intervertebral disc space. In one example, a surgeon can manipulate adjustment dial 105 to distract the superior paddle 111 from inferior paddle 112. At 425, the technique 400A can continue with the instrument detecting a pressure measurement from the force/pressure sensor disposed within one of the distraction paddles. In certain examples, the pressure measurement can be accessed by the control module 120 and/or an external computing device for additional processing or analysis. At 435, the technique 400A can continue with the user, the control module, or an external computing device determining whether a target pressure has been attained. If the target pressure (or pressure range) has been reached, the technique 400A can continue at 445, if not the technique continues back to 415 for further adjustment and on to monitor pressure measurement at 425. Determining whether the target pressure has been reached at 435 can be performed on a connected external computing device, such as a tablet via Bluetooth, monitoring the pressure measurements, which can then display the information on a user interface.

At 445, the technique 400A can continue with the user reading or a computing device receiving the distraction measurement readout. In an example utilizing an external computing device, the computing device can receive distraction measurement information from the instrument, such as via the control module 120. In another example, the distraction measurement can be read directly from the implant size indicator 106 on the implant sizing instrument 100. At 455, the technique 400A can conclude with the user or the computing device determining a recommended implant size. In an example utilizing an external computing device (or with an enhanced control module 120), the pressure measurement and distraction measurement information can be used to determine a recommended implant size. The recommended implant size can be displayed within a user interface hosted on the implant sizing instrument 100 or on an external user interface, such as user interface 200D.

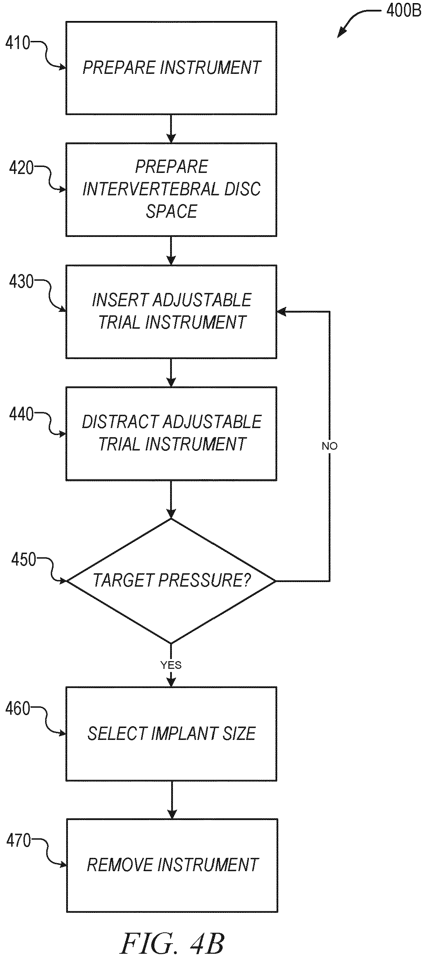

FIG. 4B is a flow chart illustrating operations of a technique 400B for determining a proper implant size using an adjustable trialing instrument, in accordance with at least one example of the disclosure. In this example, the technique 400B includes operations such as: preparing the instrument at 410, preparing the intervertebral disc space at 420, inserting the instrument 430, distracting the instrument at 440, determining if a target pressure has been reached at 450, selecting an implant size at 460, and removing the instrument at 470. While certain operations discussed within technique 400B can be performed on or enhanced by a computing device or control circuit or surgical system 300, the technique is primarily discussed in terms of an end user, such as a surgeon, performing the operations.

In an example, the technique 400B can optionally begin at 410 with preparation of the adjustable trialing instrument. Instrument preparation can include operations such a powering up the instrument, resetting or calibrating the pressure/force sensor, connecting the instrument to an external computing device (e.g., establishing a Bluetooth or similar wireless connection), and resetting the adjustable trialing mechanism 110 among other operations. At 420, the technique 400B can continue with the surgeon preparing the intervertebral disc space for an implant. Disc space preparation can include removing damaged or diseased disc material in preparation for implanting a replacement prosthetic disc, such as Zimmer Biomet's Mobi-C^{®}. After the disc space is prepared, the technique 400B can continue at 430 with insertion of an adjustable trialing instrument, such as implant sizing instrument 100. Inserting the implant sizing instrument 100 includes positioning the distraction paddles, superior paddle 111 and inferior paddle 112, in a position similar to the target implant position within the intervertebral disc space.

Once the distraction paddles are in position, the technique 400B can continue at 440 with distraction of the adjustable trialing mechanism 110. In an example, distraction of the adjustable trialing mechanism 110 includes manipulation of adjustment dial 105. At 450, the technique 400B continues with checking the intervertebral disc pressure measurement generated by the sensor within the adjustable trialing mechanism 110 to determine if the target pressure has been achieved. If the target pressure has not be reached, the technique 400B continues back to operation 430 and 440 to continue distracting the adjustable trial instrument and monitoring the pressure measurement. If the target pressure has been attained, the technique 400B can continue at 460 with selection of an implant based on sizing information provided by the implant sizing instrument 100. In an example, the implant sizing instrument 100 includes an implant size indicator 106, which provides a direct reading of recommended implant height. In other examples, a user interface displayed on an external computing device, such as user interface 200D, can provide implant sizing information based on the distraction height measurement and the pressure measurement. Technique 400B can conclude with removal of the implant sizing instrument from the intervertebral disc space.

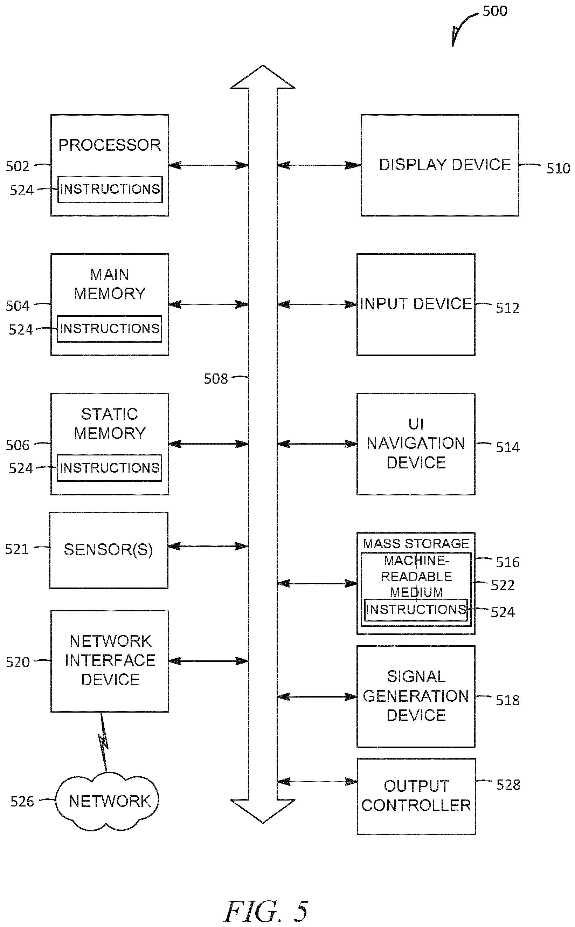

FIG. 5 illustrates a block diagram of an example machine 500 upon which any one or more of the techniques discussed herein may perform in accordance with some embodiments. The machine 500 can also host any of the user interfaces discussed herein. In alternative embodiments, machine 500 may operate as a standalone device or may be connected (e.g., networked) to other machines. In a networked deployment, machine 500 may operate in the capacity of a server machine, a client machine, or both in server-client network environments. In an example, machine 500 may act as a peer machine in peer-to-peer (P2P) (or other distributed) network environment. Machine 500 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Machine (e.g., computer system) 500 may include hardware processor 502 (e.g., a central processing unit (CPU), a graphics processing unit (GPU), a hardware processor core, or any combination thereof), main memory 504 and static memory 506, some or all of which may communicate with each other via interlink (e.g., bus) 508. Machine 500 may further include display unit 510, alphanumeric input device 512 (e.g., a keyboard), and user interface (UI) navigation device 514 (e.g., a mouse). In an example, display unit 510, input device 512 and UI navigation device 514 may be a touch screen display. Machine 500 may additionally include storage device (e.g., drive unit) 516, signal generation device 518 (e.g., a speaker), network interface device 520, and one or more sensors 521, such as a global positioning system (GPS) sensor, compass, accelerometer, or other sensor. Machine 500 may include output controller 528, such as a serial (e.g., Universal Serial Bus (USB), parallel, or other wired or wireless (e.g., infrared (IR), near field communication (NFC), etc.) connection to communicate or control one or more peripheral devices (e.g., a printer, card reader, etc.).

Storage device 516 may include machine readable medium 522 on which is stored one or more sets of data structures or instructions 524 (e.g., software) embodying or utilized by any one or more of the techniques or functions described herein. Instructions 524 may also reside, completely or at least partially, within main memory 504, within static memory 506, or within hardware processor 502 during execution thereof by machine 500. In an example, one or any combination of hardware processor 502, main memory 504, static memory 506, or storage device 516 may constitute machine readable media.

While machine readable medium 522 is illustrated as a single medium, the term "machine readable medium" may include a single medium or multiple media (e.g., a centralized or distributed database, and/or associated caches and servers) configured to store the one or more instructions 524. The term "machine readable medium" may include any medium that is capable of storing, encoding, or carrying instructions for execution by machine 500 and that cause machine 500 to perform any one or more of the techniques of the present disclosure, or that is capable of storing, encoding or carrying data structures used by or associated with such instructions. Non-limiting machine readable medium examples may include solid-state memories, and optical and magnetic media.

Instructions 524 may further be transmitted or received over communications network 526 using a transmission medium via network interface device 520 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, peer-to-peer (P2P) networks, among others. In an example, network interface device 520 may include one or more physical jacks (e.g., Ethernet, coaxial, or phone jacks) or one or more antennas to connect to communications network 526. In an example, network interface device 520 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by machine 500, and includes digital or analog communications signals or other intangible medium to facilitate communication of such software.

The systems, devices and methods discussed in the present application can be useful in performing registration processes of fiducial markers with robotic surgical systems, such as by improving the accuracy of the registration process. In particular, the systems, devices and methods described herein facilitate more precise engagement between a pointer probe tip and a fiducial marker and better recognition of proper engagement between a pointer probe tip and the fiducial marker by an operator or surgeon. Such benefits can reduce error in the registration process, which can correlate to reduced error in performing a medical procedure on a patient.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventor also contemplates examples in which only those elements shown or described are provided. Moreover, the present inventor also contemplates examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as embodiments, with each claim standing on its own as a separate embodiment, and it is contemplated that such embodiments can be combined with each other in various combinations or permutations. The scope of the invention should be determined with reference to the appended claims.

## Claims

1. An implant-trialing instrument comprising:
a handle (101) including an elongate body and an adjustment input (104); and
an adjustable implant trial (110) disposed on a distal end of the elongate body and adapted to expand within an intervertebral space in response to input from the adjustment input (104), the adjustable implant trial (110) including a measurement sensor configured to output an objective measure of intervertebral disc space correlated to implant size,
wherein the handle (101) further includes a user interface display (122a) including a series of indicator lights to display output from the measurement sensor, and
wherein the series of indicator lights are configured to display light patterns indicative of different implant sizes, or wherein the adjustment input includes an implant size indicator and the series of indicator lights are configured to display light patterns indicative of go-no go conditions related to the implant size indicator,
wherein the handle (101) includes an embedded controller circuit correlating data received from the adjustment input (104) and the measurement sensor to output information to the user interface display (122a).

2. The implant trialing instrument of claim 1, wherein the embedded controller circuit outputs implant size recommendation based on data received from the adjustment input (104) and the measurement sensor.

3. The implant trialing instrument of claim 2, wherein the implant size recommendation is based on a distraction distance of the adjustable implant trial (110) and a pressure output from the measurement sensor, the pressure output being within a pre-defined range corresponding to a range of mobility.

4. The implant trialing instrument of claim 1, wherein the embedded controller circuit includes a wireless communication interface to transmit output date to a remote computer system.

## Patentansprüche

1. Implantat-Testinstrument, umfassend:
einen Griff (101), der einen länglichen Körper und einen Einstelleingang (104) enthält; und
ein einstellbares Probeimplantat (110), das an einem distalen Ende des länglichen Körpers angeordnet ist und dafür ausgelegt ist, sich als Reaktion auf eine Eingabe von der Einstelleingabe (104) innerhalb eines Zwischenwirbelraums auszudehnen, wobei das einstellbare Probeimplantat (110) einen Messsensor enthält, der dazu konfiguriert ist, ein objektives Maß des Bandscheibenraums, das mit der Implantatgröße korreliert ist, auszugeben,
wobei der Griff (101) ferner eine Benutzerschnittstellenanzeige (122a) enthält, die eine Reihe von Anzeigeleuchten enthält, um eine Ausgabe von dem Messsensor anzuzeigen, und
wobei die Reihe von Anzeigeleuchten dazu konfiguriert ist, Lichtmuster anzuzeigen, die unterschiedliche Implantatgrößen angeben, oder wobei die Einstelleingabe eine Implantatgrößenanzeige enthält und die Reihe von Anzeigeleuchten dazu konfiguriert ist, Lichtmuster anzuzeigen, die Go-No-Go-Bedingungen in Bezug auf die Implantatgrößenanzeige angeben,
wobei der Griff (101) eine eingebettete Steuerschaltung enthält, die Daten korreliert, die von dem Einstelleingang (104) und dem Messsensor empfangen werden, um Informationen an die Benutzerschnittstellenanzeige (122a) auszugeben.

2. Implantat-Testinstrument nach Anspruch 1, wobei die eingebettete Steuerschaltung eine Implantatgrößenempfehlung basierend auf Daten ausgibt, die von dem Einstelleingang (104) und dem Messsensor empfangen werden.

3. Implantat-Testinstrument nach Anspruch 2, wobei die Implantatgrößenempfehlung auf einem Distraktionsabstand des einstellbaren Probeimplantats (110) und einer Druckausgabe des Messsensors basiert, wobei die Druckausgabe innerhalb eines vordefinierten Bereichs liegt, der einem Mobilitätsbereich entspricht.

4. Implantat-Testinstrument nach Anspruch 1, wobei die eingebettete Steuerschaltung eine drahtlose Kommunikationsschnittstelle enthält, um Ausgabedaten an ein entferntes Computersystem zu übertragen.

## Revendications

1. Instrument d'essai d'implant comprenant :
une poignée (101) comprenant un corps allongé et une entrée de réglage (104) ; et
un essai d'implant réglable (110) disposé sur une extrémité distale du corps allongé et adapté pour se dilater à l'intérieur d'un espace intervertébral en réponse à une entrée provenant de l'entrée de réglage (104), l'essai d'implant réglable (110) comprenant un capteur de mesure conçu pour délivrer en sortie une mesure objective de l'espace de disque intervertébral corrélé à la taille de l'implant,
ladite poignée (101) comprenant en outre un dispositif d'affichage d'interface utilisateur (122a) comprenant une série de voyants lumineux pour afficher une sortie du capteur de mesure, et
ladite série de voyants lumineux étant conçue pour afficher des motifs lumineux indiquant différentes tailles d'implants, ou ladite entrée de réglage comprenant un indicateur de taille d'implant et ladite série de voyants lumineux étant conçue pour afficher des motifs lumineux indiquant des conditions tout ou rien liées à l'indicateur de taille d'implant,
ladite poignée (101) comprenant un circuit de dispositif de commande incorporé corrélant les données reçues en provenance de l'entrée de réglage (104) et du capteur de mesure pour délivrer en sortie des informations au dispositif d'affichage d'interface utilisateur (122a).

2. Instrument d'essai d'implant selon la revendication 1, ledit circuit de dispositif de commande incorporé délivrant en sortie une recommandation de taille d'implant sur la base des données reçues en provenance de l'entrée de réglage (104) et du capteur de mesure.

3. Instrument d'essai d'implant selon la revendication 2, ladite recommandation de taille d'implant étant basée sur une distance de distraction de l'essai d'implant réglable (110) et une sortie de pression en provenance du capteur de mesure, la sortie de pression étant dans les limites d'une plage prédéfinie correspondant à une plage de mobilité.

4. Instrument d'essai d'implant selon la revendication 1, ledit circuit de dispositif de commande incorporé comprenant une interface de communication sans fil pour transmettre la date de sortie à un système informatique à distance.
